# EUROPEAN PATENT SPECIFICATION

(11) **EP 4 272 165 B1**
(45) Date of publication and mention of the grant of the patent: **31.12.2025**
(21) Application number: 21844963.5
(22) Date of filing: 21.12.2021
(51) Int. Cl.: G06T 7/90, G06T 7/11, G06T 7/194, G06T 11/00, A45D 34/04, A45D 34/06, A45D 44/00, B08B 3/08, G06Q 30/0601, G06V 10/56, G06V 10/82, H04W 4/80, H04W 64/00, A45D 34/00, G06Q 50/00

(54) **METHOD FOR DETERMINING AT LEAST ONE COLOUR COMPATIBLE WITH AN OUTFIT OF A USER**
VERFAHREN ZUR BESTIMMUNG MINDESTENS EINER ZUR KLEIDUNG EINES BENUTZERS PASSENDEN FARBE
MÉTHODE POUR DÉTERMINER AU MOINS UNE COULEUR COMPATIBLE AVEC UNE TENUE D'UN UTILISATEUR

(30) Priority: 31.12.2020 US 202017139340; 31.12.2020 US 202017139457; 31.12.2020 US 202017139338; 31.12.2020 US 202017139391; 31.12.2020 US 202017139454; 04.08.2021 FR 2108455
(43) Date of publication of application: 08.11.2023
(73) Proprietor: L'OREAL, 75008 Paris (FR)
(72) Inventor: JAMES, Tiffany, 92110 Clichy (FR); CHARRAUD, Grégoire, 92110 Clichy (FR); SAWANTO, Aldina, 92110 Clichy (FR)
(74) Representative: Casalonga
(86) International application number: PCT/EP2021/086978
(87) International publication number: WO 2022/144232

(56) References cited:
- WO-A1-2008/108760
- WO-A1-2012/065037
- US-A1- 2013 083 999
- US-B1- 10 109 051
- US-B1- 10 402 648
- CHUNG KYUNG-YONG: "Effect of facial makeup style recommendation on visual sensibility", MULTIMEDIA TOOLS AND APPLICATIONS, KLUWER ACADEMIC PUBLISHERS, BOSTON, US, vol. 71, no. 2, 7 February 2013 (2013-02-07), pages 843 - 853, XP035307406, ISSN: 1380-7501, [retrieved on 20130207], DOI: 10.1007/S11042-013-1355-6

## Description

Modes of implementation and embodiments relate to determining one or more colours compatible with an outfit of a user, for example in the context of assisting with or providing recommendations with respect to a choice of a cosmetic make-up product.

By colour "compatible" with the outfit, what is meant is a colour that is in aesthetic agreement, that is in harmony, with the colours of the outfit.

Services, provided by individuals (make-up artists) who are qualified in the art of colour compatibility, exist whereby combinations of compatible colours, i.e. colours that go well together according to generally accepted criteria, subjective criteria or even fashion-related criteria, are recommended.

Because of the subjective aspect of this type of recommendation, it is difficult to provide automatic techniques for determining colours compatible with an outfit, without the outfit being known in advance.

However, there is a need to provide, depending on a given outfit, the outfit changing each time, recommendations as to compatible colours to consumers of make-up who are not qualified make-up artists, immediately (i.e. without consultation, by appointment, of a person qualified in the art).

The recommendations as to compatible colours may thus be used to assist consumers of make-up in making an aesthetic choice, or even potentially be used to obtain a product having this colour compatible with the outfit.

Specifically, new "connected" methods for consuming cosmetic products are emerging and for example make it possible to obtain, at home, a cosmetic product the recipe of which is unique and manufactured by a device controlled by a smartphone. This is in particular the case of the cosmetic-product distributor described in US patent US 12,033,202 B2 and in US patent application US 2021/0235849 A1.

Thus, a determined colour compatible with a given outfit may be used, every day, to generate, instantaneously and on a daily basis, the cosmetic product having precisely this compatible colour.

WO 2008/108760 A1, Hewlett Packard Development Co. describes a technique for determining a user's skin colour from a photographic image, in particular in order to offer a recommendation of cosmetic products, or products related to personal appearance, in colours that are suitable for the determined skin colour.

US 10 402 648 B1, Shimozaki describes colour analysis of items worn by a user, in particular: determining a portion of a wearable item identified within an image as being a patch portion representative of the wearable item depicted within the image by a processor. Patterns of the wearable item are derived by the processor based on image analysis of the determined patch portion of the image. Colours of the wearable item are derived by the processor based on image analysis of the determined patch portion of the image. Information regarding the derived colours and information regarding the derived patterns are transmitted.

In this respect, according to one aspect a method is provided for automatically determining, within a computational system, at least one colour of a cosmetic product, for example a cosmetic make-up product, compatible with an outfit of a user, comprising:
- a step of importing at least one photographic image containing a representation of at least one portion of the outfit of the user;
- a step of segmenting said at least one image, to identify at least one region of interest in the outfit;
- a step of determining the colour of said at least one region of interest; and
- a step of generating said at least one colour compatible with the outfit of the user, on the basis of the determined colour of said region of interest depending on a database of compatible colours or on a computational rule for obtaining compatible colours.

The computational system may for example be a user computational device alone, such as a smartphone, a touch-screen tablet or a computer, alone, or indeed a user computational device and an external server that are able to communicate and interact with each other.

The method according to this aspect may thus be implemented automatically, for example by means of a single user computational device such as a smartphone, to determine colours compatible with an outfit, without the outfit being known in advance.

The method may thus provide the user with one or more colours for their make-up depending on their clothes, and optionally on their preferences.

Furthermore, the method may make it possible to recommend purchase of complete make-up products based on the association of the colours, or indeed to order an "ideal" recipe for each outfit, this recipe being manufactured by a personal device such as the aforementioned connected cosmetic-product distributor.

According to one mode of implementation, the generating step, carried out depending on a database of compatible colours, comprises:
- a step of identifying, among banks of colours containing a finite number of colours chosen beforehand to be compatible with one another, the bank of colours containing the colour closest to the colour of said determined region of interest; and
- a step of selecting said at least one colour compatible with the outfit from the colours of the identified bank.

According to one mode of implementation, the generating step, carried out depending on a computational rule for obtaining compatible colours, comprises:
- a computing step comprising converting the colour of said determined region of interest into a first point in a suitable colour space, and applying at least one mathematical transformation to this point, said at least one mathematical transformation being chosen beforehand to obtain as result at least one second point in said space the colour of which is compatible with the colour of the first point; and
- a step of selecting said at least one colour compatible with the outfit from the one or more colours of said at least one second point.

According to one mode of implementation, the selecting step comprises selecting said at least one compatible colour from the obtained colours, these belonging to a family of colours selected by the user.

The colour family may correspond to a preference of the user, or indeed to a family of possible recipes given the cartridges loaded into a personal device such as the aforementioned connected cosmetic-product distributor.

According to one mode of implementation, the generating step is implemented using a machine-learning model.

Advantageously, the machine-learning model is configured to tailor the selecting step depending on prior choices of the user as to compatible colours determined in prior implementations of the method.

The machine-learning model may for example be a convolutional neural network, and makes it possible to benefit from an extremely fine precision in the generation of the colour compatible with the outfit, this being particularly advantageous in this context of colour harmony and of aestheticism.

According to one mode of implementation, the segmenting step is implemented automatically using a machine-learning model.

The machine-learning model may once again be a convolutional neural network, and makes it possible on the one hand to avoid errors due to segmentation of an irrelevant portion (for example a body part or part of the background), and on the other hand to detect advantageous details of the outfit, such as the colours of accessories or jewellery or small patterns on the outfit, that are too delicate for techniques not employing machine learning to detect.

According to one mode of implementation, the segmenting step comprises a request addressed to the user to identify the position of said at least one region of interest in the image.

According to one mode of implementation, the determining step comprises carrying out colour processing on said at least one image using a machine-learning model suitable for correcting a deformation of the colour of the representation of said at least one portion of the outfit of the user due to the lighting conditions of the photograph and/or to the device having captured the photograph.

For example, the machine-learning model suitable for correcting a deformation of colour may be such as that described in French patent application filed 03 February 2021 under number FR2101039.

According to another aspect, a computational system, for example a user computational device alone or indeed a user computational device and an external server that are able to communicate and interact with each other, is provided, said system being intended to determine at least one colour of a cosmetic product compatible with an outfit of a user, and comprising:
- communication means configured to import at least one photographic image containing a representation of at least one portion of the outfit of the user;
- processing means configured to:
   -- segment said at least one image, so as to identify at least one region of interest in the outfit;
   -- determine the colour of said at least one region of interest; and
   -- generate said at least one colour compatible with the outfit of the user, on the basis of the determined colour of said region of interest depending on a database of compatible colours or on a computational rule for obtaining compatible colours.

According to one embodiment, the processing means are further configured to implement the segmenting steps, the determining step and the generating step of the method such as defined above.

According to one embodiment, the system comprises a user computational device configured to capture or store in memory said at least one photographic image and to communicate to the processing means said at least one photographic image in said importing step.

According to another aspect, a computer program product is also provided, said product comprising instructions that, when the program is executed by a computer, cause the latter to implement the method such as defined above.

According to another aspect, a computer-readable medium is also provided, said medium comprising instructions that, when they are executed by a computer, cause the latter to implement the method such as defined above.

Other advantages and features of the invention will become apparent on examining the detailed description of modes of implementation and embodiments, which are in no way limiting, and the appended drawings, in which:
[Fig.1];

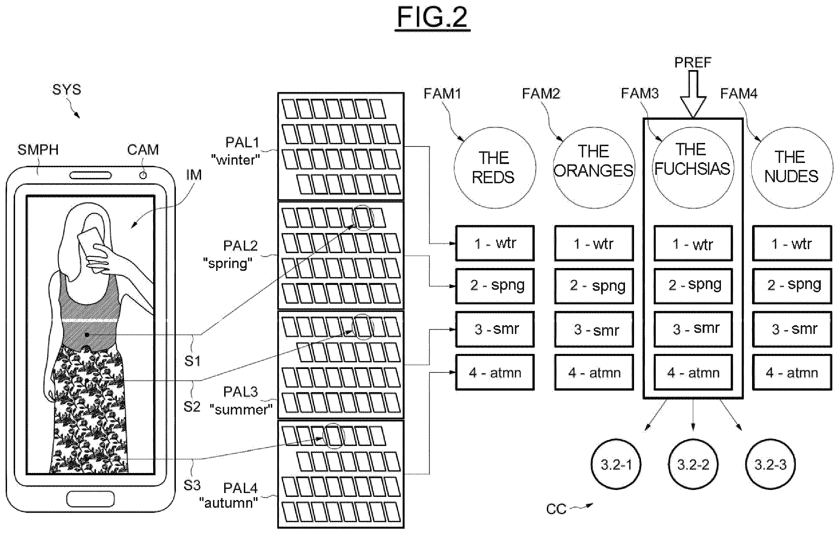

[Fig.2] illustrate modes of implementation and embodiments of the invention.

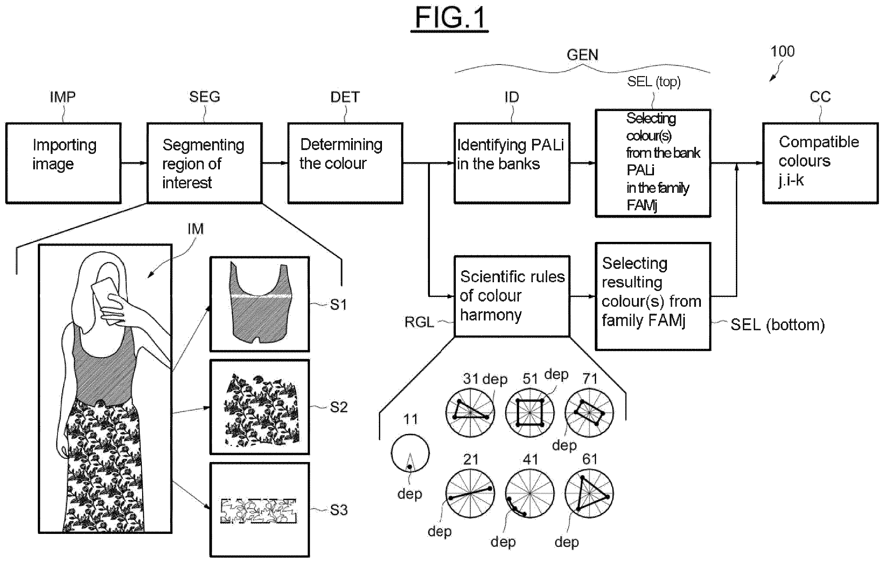

Figure 1 illustrates one example of implementation of a method 100 for determining at least one colour CC compatible with an outfit of a user.

The method 100 is for example intended to be implemented by a computational device, such as a smartphone, a touch-screen tablet, or a computer. Optionally, the method 100 may be implemented in collaboration with an external server in a configuration in which the server receives input data (image IM), executes at least certain of the operations of the steps of the method 100 (among SEG, DET, GEN, SEL), and delivers output data (CC).

Thus, the method 100 may in practice be embodied by a computer program product comprising instructions that, when the program is executed by a computer, cause the latter to implement the method 100.

The method 100 may also in practice be embodied in the form of a computer-readable medium comprising instructions that, when they are executed by a computer, cause the latter to implement the method 100.

The method 100 for determining at least one colour CC compatible with an outfit of a user may for example be offered as a service to the user with a view to providing, depending on a given outfit, the outfit changing each time, them with recommendations as to colours, of make-up for example, that are compatible, from an aesthetic point of view, immediately (i.e. without consultation, by appointment, of a make-up artist qualified in the art).

The recommendations as to compatible colours may thus be used to assist consumers of make-up who are not qualified make-up artists in making an aesthetic choice, or even potentially be used to obtain one or more commercially available products having the one or more colours compatible with the outfit.

Specifically, the method 100 may furthermore be used to recommend purchase of complete make-up products based on the association of the colours, or indeed to order an "ideal" recipe for each outfit, this recipe being manufactured by a personal device such as a connected cosmetic-product distributor.

In this respect, the method 100 comprises a step IMP of importing at least one photographic image IM containing a representation of at least one portion of the outfit of the user.

For example, the step IMP of importing the photographic image may result from a capture performed by the user, in selfie mode or optionally by photographing their reflection in a mirror.

Next, the method 100 comprises a step of segmenting SEG said at least one image IM, so as to identify at least one region of interest S1, S2, S3 in the outfit.

The segmenting step SEG may be implemented automatically using an image-segmenting algorithm that is for example configured to recognize objects, define contours, recognize patterns, remove background, or carry out other suitable and known image-processing operations.

In particular, the segmenting step SEG is configured to identify elements, called "regions of interest", S1, S2, S3 of the outfit present in the image IM.

The segmenting step SEG is for example capable of detecting an element S1 of the top portion of the outfit, close to the face, such as a blouse, a T-shirt or a bodysuit.

The segmenting step SEG is for example capable of detecting an element S2 of the middle portion of the outfit, close to the pelvis, such as a pair of trousers, a skirt or a dress.

The segmenting step SEG is for example capable of detecting an element S3 of the bottom portion of the outfit, close to the feet, such as shoes, socks or the bottom of a pair of trousers, of a skirt or of a dress.

Of course, the elements S1, S2, S3 identified in the image depend on the way in which the photo was captured, and for example shoes may not feature in the image IM.

In the example illustrated in Figure 1, the middle region of interest S2 and the bottom region of interest S3 both correspond to a portion of a long skirt. In such a situation, the segmenting step SEG is for example configured to identify and isolate a pattern and to remove the background colour of the skirt in the region of interest S2, and to identify and isolate the background colour of the skirt and to remove the pattern in the region of interest S3.

Moreover, the segmenting step SEG may be implemented automatically with a conventional machine-learning model such as, for example, a convolutional neural network. This makes it possible on the one hand to avoid errors due to segmentation of an irrelevant portion (for example a body part or part of the background), and on the other hand to detect advantageous details of the outfit, such as the colours of accessories or jewellery or small patterns on the outfit, that are too delicate for techniques not employing machine learning to detect.

Alternatively, as will be described below with reference to Figure 2, the segmenting step SEG may be performed "manually" by the user, for example by means of a request addressed to the user to identify the position of said at least one region of interest in the image IM.

On the basis of the regions of interest S1, S2, S3 identified in the segmenting step SEG, the method 100 comprises a step DET of determining the colour of the regions of interest S1, S2, S3.

The colour-determining step DET may for example extract the dominant colour of each region of interest S1, S2, S3, or optionally average the colours present in each region of interest S1, S2, S3.

Advantageously, the determining DET step comprises applying colour processing to the image IM, said processing being suitable for correcting a deformation of the colour in the image caused by the lighting conditions of the photograph and/or by the device having captured the photograph.

Specifically, the colour may be deformed depending on the nature of the device having captured the photograph. There are methods for compensating the deformation due to a given piece of hardware.

Furthermore, the lighting conditions may also modify the perception of a colour: typically the colour will have a colder colour temperature if the lighting conditions, i.e. the sources of light in the photographed scene, are cold; and the colour will have a warmer colour temperature if the lighting conditions are warm.

A machine-learning model may advantageously be provided to correct a deformation of the colour, for example taking into account the geographic position of the image capture (inside or outside), the timestamp of the image capture (day or night), the weather at the time of the image capture (sunny, cloudy, raining, snowing). The parameters allowing the correction to be estimated may be more complex than the basic elements presented above.

The machine-learning model may for example be a convolutional neural network. Use of a machine-learning model makes it possible to benefit from an extremely fine precision in the determination DET of the colour of the regions of interest S1, S2, S3 of the outfit of the user, this being particularly advantageous in this context of colour harmony and of aestheticism.

After the "actual" colours of the regions of interest S1, S2, S3 have been determined DET, the method 100 comprises a step GEN of generating at least one colour CC compatible with the outfit of the user.

The generation GEN is carried out on the basis of the one or more "actual" colours determined in the determining step DET.

In this respect, two approaches ID-SEL, RGL-SEL are possible.

The first approach RGL-SEL may use "the seven rules of the science of colour and of harmony" to target the formation of a certain type of relationship between the colours of the outfit and the compatible colours.

In this first approach RGL-SEL, the generating step GEN is carried out depending on a computational rule RGL for obtaining compatible colours, and comprises a computing step RGL followed by a selecting step SEL.

The computing step RGL comprises converting the determined colour of the region of interest into a first point "dep" of a suitable colour space, for example the "HSV" space, and applying at least one mathematical transformation 11, 21, 31, 41, 51, 61, 71 to this "starting" point dep.

The mathematical transformation is chosen beforehand, according to the seven rules of the science of colour and of harmony, to obtain as a result at least one second point in said space the colour of which is compatible with the colour of the first point dep.

The seven rules of the science of colour and of harmony are defined, in the "HSV" or "hue-saturation-value" colour space, which is well suited to the characterization of colours by human beings, in the following way:
The first rule 11 is a monochromatic transformation, i.e. a transformation to various saturations and values of the same hue as the starting colour dep.
The second rule 21 is a complementary transformation of the starting colour dep, i.e. a projection to the hue radially opposite the hue of the starting colour in a representation of the hues on a disc of revolution.
The third rule 31 is an adjacent complementary transformation, i.e. a projection to hues neighbouring the hue complementary to the starting colour dep.
The fourth rule 41 is a transformation to adjacent colours, i.e. a projection to hues neighbouring the hue of the starting colour dep.
The fifth rule 51 is a quadratic transformation, i.e. a projection of three hues forming, with the starting hue dep, a square centred on the disc of resolution of the hues.
The sixth rule 61 is a triadic transformation, i.e. a projection of two hues forming, with the starting hue dep, an isosceles triangle centred on the disc of resolution of the hues.
The seventh rule 71 is a rectangular transformation, i.e. a projection of three hues forming, with the starting hue dep, a rectangle centred on the disc of resolution of the hues.

Advantageously, in all the rules 21-71 except the monochromatic first rule 11, the saturation and value of the starting colour will be preserved, i.e. remain the same, in the colours obtained by the various transformations 21-71.

On the basis of the colours obtained via the various transformations 11-71, the generation GEN of the compatible colour CC comprises selecting SEL at least one compatible colour CCD (j.i-k) from the one or more resulting colours.

For example, the resulting colours may be selected depending on their membership of a colour family chosen by the user. For example, in the context of make-ups such as foundation or lipstick, the colour families may be "the reds", "the oranges", "the fuchsias" or "the nudes".

The colour family may correspond to a preference PREF of the user (figure 2), or indeed to a family of possible recipes given the cartridges loaded into a personal device such as a connected cosmetic-product distributor.

Moreover, the generating step GEN may be implemented with a machine-learning model configured and trained to select the colours, resulting from the seven rules of the science of colour and of harmony, that are best suited to a given type of make-up.

Furthermore, the machine-learning model is advantageously configured to tailor the selecting step SEL depending on prior choices of the user as to compatible colours determined in prior implementations of the method 100.

Once again, the machine-learning model may for example be a convolutional neural network. Once again, use of a machine-learning model makes it possible to benefit from an extremely fine precision in the generation of the colour CC compatible with the outfit, this being particularly advantageous in this context of colour harmony and of aestheticism.

The second approach ID-SEL may use a palette that has been predetermined on the basis of a recommendation of a make-up artist in light of a seasonal style in combination with the colour of the outfit.

In this regard, reference is made to Figure 2.

Figure 2 illustrates the method 100 in the context of the second approach ID-SEL to the step of generating the compatible colours CC.

In this second approach ID-SEL, the generating step GEN is carried out depending on a database PALi of compatible colours, 1 ≤ i ≤ 4, and comprises an identifying step ID followed by a selecting step SEL.

Banks, or "pallets", PAL1, PAL2, PAL3, PAL4, of colours, each containing a finite number of colours are defined beforehand by a make-up artist so as to contain colours that are aesthetically compatible with one another.

For example, each pallet PALi, 1 ≤ i ≤ 4, may correspond to a seasonal style: "winter", "spring", "summer", "autumn".

The identifying step ID (Figure 1) comprises identifying, among the banks PAL1, PAL2, PAL3, PAL4 of colours the bank PALi of colours, 1 ≤ i ≤ 4, containing the colour closest to the colour of said determined region of interest S1, S2, S3.

On the basis of the colours obtained via the various transformations 11-71, the generation GEN of the compatible colour CC comprises selecting SEL at least one compatible colour CC (j.i-k, for example j=3, i=2, k=1 or 2 or 3) from the colours of the one or more identified banks PALi, 1 ≤ i ≤ 4.

For example, the resulting colours may be selected depending on their membership of a colour family FAMj, 1 ≤ j ≤ 4, chosen PREF by the user. For example, in the context of make-ups such as foundation or lipstick, the colour families may be "the reds", "the oranges", "the fuchsias" or "the nudes".

The colour family FAMj, 1 ≤ j ≤ 4, may correspond to a preference PREF of the user 2, or indeed to a family of possible recipes given the cartridges loaded into a personal device such as a connected cosmetic-product distributor.

Thus, a compatible colour CC may be identified by an obtained pair j.i in a table on a row "i" corresponding to the identified palette PALi, 1 ≤ i ≤ 4, and in a column "j" corresponding to the chosen family FAMj, 1 ≤ j ≤ 4. A plurality of compatible colours CC may be selected for a given pair j.i, each then being identified by an additional index "k". For example the three compatible colours 3.2-1, 3.2-2, 3.2-3, i.e. j=3, i=2, k=1 or 2 or 3, are obtained from the family FAM3 "the fuchsias" in the "spring" palette PAL2.

When a plurality of pallets PALi, 1 ≤ i ≤ 4, are identified for a plurality of regions of interest S1, S2, S3, respectively, then priority is advantageously given to the region of interest S1 closest to the face of the user in the image IM.

Therefore, for a chosen family FAMj, j=J, if a single palette PALi, i=I, is identified, then the selecting step SEL delivers K compatible colours J.I-k, 1 ≤ k ≤ K, with for example K=3.

For a chosen family FAMj, j=J, if two pallets PALi, i=I1 or I2, are identified, then the selecting step SEL delivers K1 compatible colours J1.I1-k, 1 ≤ k ≤ K1; and K2 compatible colours J1.I2-k, 1 ≤ k ≤ K2, with K1+K2=K and K2 ≤ K1, with for example K1=2 and K2=1.

For a chosen family FAMj, j=J, if three pallets PALi, i=I1 or I2 or I3, are identified, then the selecting step SEL delivers K1 compatible colours J1.I1-k, 1 ≤ k ≤ K1; K2 compatible colours J1.I2-k, 1 ≤ k ≤ K2; and K3 compatible colours J1.I3-k, 1 ≤ k ≤ K3, with K1+K2+K3=K and K3 ≤ K2 ≤ K1, with for example K1=1, K2=1 and K3=1.

Moreover, the generating step may be implemented with a machine-learning model configured and trained to generate colours that belong to the given palettes PALi, and that are best suited to a given type of make-up.

Furthermore, the machine-learning model is advantageously configured to tailor the selecting step SEL depending on prior choices of the user as to compatible colours determined in prior implementations of the method 100.

Once again, the machine-learning model may for example be a convolutional neural network. Once again, use of a machine-learning model makes it possible to benefit from an extremely fine precision in the generation of the colour CC compatible with the outfit, this being particularly advantageous in this context of colour harmony and of aestheticism.

Moreover, as an alternative to the automatic segmentation SEG described with reference to Figure 1, Figure 2 illustrates an example of segmentation SEG performed "manually" by the user.

Specifically, the user may position probes S1, S2, S3 on his choice of regions of interest of the outfit, for example by virtue of a touchscreen displaying the image IM. From that point of view of the method 100, which is for example executed by a smartphone SMPH, the segmenting step SEG comprises a request addressed to the user to identify the position of said at least one region of interest S1, S2, S3 in the image IM.

Thus, figure 2 illustrates a computational system SYS intended to determine at least one colour compatible with an outfit of a user, and suitable for implementing the method 100 described above.

The system SYS includes a user computational device, for example the smartphone SMPH, and optionally an external server.

The user computational device SMPH comprises communication means configured to perform the importing step IMP of the method 100, for example via the Internet and with communication means of the server.

For example, the user computational device SMPH typically comprises a photographic sensor CAM configured to capture the photographic image IM, and/or typically a memory suitable for storing said at least one photographic image IM.

The external server, or alternatively the user computational device SMPH, comprises processing means configured to perform the segmenting step SEG of the method 100, the determining step DET of the method 100, and the step GEN of generating the compatible colours CC.

When the processing means are all incorporated into the user computational device SMPH, the importing step corresponds to an internal transfer of the data of the image IM, from the photographic sensor CAM or from the memory, to the processing means.

In other words, a method 100 and a corresponding system that, using the camera CAM of a telephone SMPH, extract the colour of certain bits ("regions of interest") of the outfit (for example a blouse, a pair of trousers or of shoes), have been described.

With this set of colours, the method 100 then classes each thereof in a "universe" or a specific "season" (summer, winter, spring, autumn) by finding the closest colour match in the pre-existing banks PAL1-PAL4 of colours and their corresponding colour universes.

Furthermore, a "sub-selection" is made in a colour family FAMj desired PREF by the user to achieve even greater precision in the hues of the colours.

Make-up products in a given universe PALi and optionally a given family FAMj of colours may then be recommended, with priority being given to the colours closest to the face, in order to optimize the coordination of the make-up of the user with their outfit.

The choice of the family FAMj may correspond to the colours that a connected make-up distributor is able to produce, on the basis of the set of cartridges installed in the distributor and on the basis of the number and type of cartridges of the distributing device, and on the basis of the number and priority of the probes that the user has decided to use. The user may also choose another family FAMj in order to view options that would be available with other sets of cartridges. This may incite the user to buy a new set of cartridges.

Furthermore, use of artificial intelligence makes it possible to offer the user a new type of hue-recommending service. By adopting a more inclusive approach taking into account other elements of their everyday outfits, such as their clothes, their shoes and their accessories, the recommendations may be frequently tailored to the preferences of the user.

## Claims

1. Method for automatically determining, within a computational system, at least one colour of a cosmetic product compatible with an outfit of a user, comprising:
- a step (IMP) of importing at least one photographic image (IM) containing a representation of at least one portion of the outfit of the user;
- a step (SEG) of segmenting said at least one image (IM), to identify at least one region of interest (S1, S2, S3) in the outfit;
- a step (DET) of determining the colour of said at least one region of interest (S1, S2, S3); and
- a step (GEN) of generating said at least one colour compatible with the outfit of the user, on the basis of the determined colour of said region of interest (S1, S2, S3) depending on a database (PAL1-PAL4) of compatible colours or on a computational rule (RGL, 11-71) for obtaining compatible colours.

2. Method according to Claim 1, wherein the generating step (GEN), carried out depending on a database of compatible colours (PAL1-PAL4), comprises:
- a step (ID) of identifying, among banks (PAL1-PAL4) of colours containing a finite number of colours chosen beforehand to be compatible with one another, the bank (PAL1) of colours containing the colour closest to the colour of said determined region of interest (S1, S2, S3); and
- a step (SEL) of selecting said at least one colour (c3.1, c3.2, c3.3) compatible with the outfit from the colours of the identified bank (PAL1).

3. Method according to either of Claims 1 and 2, wherein the generating step (GEN), carried out depending on a computational rule (RGL, 11-71) for obtaining compatible colours, comprises:
- a computing step (RGL) comprising converting the colour of said determined region of interest (S1, S2, S3) into a first point in a suitable colour space, and applying at least one mathematical transformation (11-71) to this point, said at least one mathematical transformation (11-71) being chosen beforehand to obtain as a result at least one second point in said space the colour of which is compatible with the colour of the first point; and
- a step (SEL) of selecting said at least one colour (c3.1, c3.2, c3.3) compatible with the outfit from the one or more colours of said at least one second point.

4. Method according to either of Claims 2 and 3, wherein said selecting step (SEL) comprises selecting said at least one compatible colour from the obtained colours, these belonging to a family (FAMa-FAMd) of colours selected by the user.

5. Method according to one of the preceding claims, wherein the generating step (GEN) is implemented using a machine-learning model (AI_GEN).

6. Method according to Claim 5 in combination with one of Claims 2 to 4, wherein the machine-learning model (AI_GEN) is configured to tailor the selecting step (SEL) depending on prior choices of the user as to compatible colours determined in prior implementations of the method.

7. Method according to one of Claims 1 to 6, wherein the segmenting step (SEG) is implemented automatically using a machine-learning model (AI_TCOL).

8. Method according to one of Claims 1 to 6, wherein the segmenting step (SEG) comprises a request addressed to the user to identify the position of said at least one region of interest in the image.

9. Method according to one of the preceding claims, wherein the determining step (DET) comprises carrying out colour processing (TCOL) on said at least one image (IMk) using a machine-learning model (AI_TCOL) suitable for correcting a deformation of the colour of the representation of said at least one portion of the outfit of the user due to the lighting conditions of the photograph and/or to the device (CAM) having captured the photograph.

10. Computational system intended to determine at least one colour of a cosmetic product compatible with an outfit of a user, comprising:
- communication means (COM) configured to import at least one photographic image (IM) containing a representation of at least one portion of the outfit of the user;
- processing means (PU) configured to:
-- segment (SEG) said at least one image (IM), so as to identify at least one region of interest (S1, S2, S3) in the outfit;
-- determine (DET) the colour of said at least one region of interest (S1, S2, S3); and
-- generate (GEN) said at least one colour compatible with the outfit of the user, on the basis of the determined colour of said region of interest (S1, S2, S3) depending on a database (PAL1-PAL4) of compatible colours or on a computational rule (RGL, 11-71) for obtaining compatible colours.

11. System according to Claim 10, wherein the processing means (PU) are further configured to implement the segmenting steps (SEG), the determining step (DET) and the generating step (GEN) according to one of Claims 2 to 9.

12. System according to either of Claims 10 and 11, comprising a user computational device (APP) configured to capture or store in memory said at least one photographic image (IM) and to communicate to the processing means (PU) said at least one photographic image in said importing step (IMP).

13. Computer program product comprising instructions that, when the program is executed by a computer, cause the latter to implement the method according to one of Claims 1 to 9.

14. Computer-readable medium comprising instructions that, when they are executed by a computer, cause the latter to implement the method according to one of Claims 1 to 9.

## Patentansprüche

1. Verfahren zum automatischen Ermitteln, innerhalb eines Computersystems, mindestens einer Farbe eines kosmetischen Produkts, das mit einem Outfit eines Benutzers kompatibel ist, umfassend:
- einen Schritt (IMP) eines Importierens mindestens eines fotografischen Bildes (IM), das eine Darstellung von mindestens einem Teil des Outfits des Benutzers enthält;
- einen Schritt (SEG) eines Segmentierens des mindestens einen Bildes (IM), um mindestens ein interessierendes Gebiet (S1, S2, S3) in dem Outfit zu identifizieren;
- einen Schritt (DET) eines Bestimmens der Farbe des mindestens einen interessierenden Gebiets (S1, S2, S3); und
- einen Schritt (GEN) eines Erzeugens der mindestens einen Farbe, die mit dem Outfit des Benutzers kompatibel ist, auf der Grundlage der ermittelten Farbe des interessierenden Gebiets (S1, S2, S3) in Abhängigkeit von einer Datenbank (PAL1-PAL4) von kompatiblen Farben oder einer Rechenregel (RGL, 11-71) für ein Erhalten von kompatiblen Farben.

2. Verfahren nach Anspruch 1, wobei der Erzeugungsschritt (GEN), der in Abhängigkeit von einer Datenbank von kompatiblen Farben (PAL1-PAL4) ausgeführt wird, umfasst:
- einen Schritt (ID) eines Identifizierens aus den Farbbanken (PAL1-PAL4), die eine endliche Anzahl von zuvor gewählten Farben enthalten, die miteinander kompatibel sind, der Farbbank (PAL1), welche die Farbe enthält, die der Farbe des ermittelten interessierenden Gebiets (S1, S2, S3) am nächsten ist; und
- einen Schritt (SEL) eines Auswählens der mindestens einen Farbe (c3.1, c3.2, c3.3), die mit dem Outfit kompatibel ist, aus den Farben der identifizierten Bank (PAL1).

3. Verfahren nach einem der Ansprüche 1 oder 2, wobei der Erzeugungsschritt (GEN), der in Abhängigkeit von einer Rechenregel (RGL, 11-71) zum Erhalten kompatibler Farben ausgeführt wird, umfasst:
- einen Berechnungsschritt (RGL), der ein Umwandeln der Farbe des ermittelten interessierenden Gebiets (S1, S2, S3) in einen ersten Punkt in einem geeigneten Farbraum und ein Anwenden mindestens einer mathematischen Transformation (11-71) auf diesen Punkt umfasst, wobei die mindestens eine mathematische Transformation (11-71) zuvor gewählt wird, um als Ergebnis mindestens einen zweiten Punkt in dem Raum zu erhalten, dessen Farbe mit der Farbe des ersten Punkts kompatibel ist; und
- einen Schritt (SEL) eines Auswählens der mindestens einen Farbe (c3.1, c3.2, c3.3), die mit dem Outfit kompatibel ist, aus der einen oder den mehreren Farben des mindestens einen zweiten Punkts.

4. Verfahren nach einem der Ansprüche 2 oder 3, wobei der Auswahlschritt (SEL) ein Auswählen der mindestens einen kompatiblen Farbe aus den erhaltenen Farben umfasst, wobei diese zu einer Farbfamilie (FAMa-FAMd) gehören, die von dem Benutzer ausgewählt wird.

5. Verfahren nach einem der vorhergehenden Ansprüche, wobei der Erzeugungsschritt (GEN) unter Verwendung eines Maschinenlernmodells (AI_Gen) implementiert wird.

6. Verfahren nach Anspruch 5 in Kombination mit einem der Ansprüche 2 bis 4, wobei das Maschinenlernmodell (AI_Gen) konfiguriert ist, um den Auswahlschritt (SEL) in Abhängigkeit von vorherigen Wahlmöglichkeiten des Benutzers hinsichtlich kompatibler Farben, die in vorherigen Implementierungen des Verfahrens ermittelt wurden, maßgeschneidert anzupassen.

7. Verfahren nach einem der Ansprüche 1 bis 6, wobei der Segmentierungsschritt (SEG) automatisch unter Verwendung eines Maschinenlernmodells (AI_TCOL) implementiert wird.

8. Verfahren nach einem der Ansprüche 1 bis 6, wobei der Segmentierungsschritt (SEG) eine Anforderung umfasst, die an den Benutzer gerichtet ist, um die Position des mindestens einen interessierenden Gebiets in dem Bild zu identifizieren.

9. Verfahren nach einem der vorhergehenden Ansprüche, wobei der Ermittlungsschritt (DET) ein Ausführen einer Farbverarbeitung (TCOL) an dem mindestens einen Bild (IMk) unter Verwendung eines Maschinenlernmodells (AI_TCOL) umfasst, das geeignet ist zum Korrigieren einer Verfälschung der Farbe in der Darstellung des mindestens einen Teils des Outfits des Benutzers aufgrund der Beleuchtungsbedingungen der Fotografie und/oder der Vorrichtung (CAM), welche die Fotografie aufgenommen hat.

10. Computersystem, das dafür vorgesehen ist, mindestens eine Farbe eines kosmetischen Produkts zu ermitteln, das mit einem Outfit eines Benutzers kompatibel ist, umfassend:
- ein Kommunikationselement (COM), das konfiguriert ist zum Importieren mindestens eines fotografischen Bildes (IM), das eine Darstellung mindestens eines Teils des Outfits des Benutzers enthält;
- ein Verarbeitungselement (PU), das konfiguriert ist zum:
-- Segmentieren (SEG) des mindestens einen Bildes (IM), um mindestens ein interessierendes Gebiet (S1, S2, S3) in dem Outfit zu identifizieren;
-- Ermitteln (DET) der Farbe des mindestens einen interessierenden Gebiets (S1, S2, S3); und
-- Erzeugen (GEN) der mindestens einen Farbe, die mit dem Outfit des Benutzers kompatibel ist, auf der Grundlage der ermittelten Farbe des interessierenden Gebiets (S1, S2, S3) in Abhängigkeit von einer Datenbank (PAL1-PAL4) von kompatiblen Farben oder einer Rechenregel (RGL, 11-71) für ein Erhalten von kompatiblen Farben.

11. System nach Anspruch 10, wobei das Verarbeitungselement (PU) ferner konfiguriert ist zum Implementieren der Segmentierungsschritte (SEG), des Ermittlungsschritts (DET) und des Erzeugungsschritts (GEN) nach einem der Ansprüche 2 bis 9.

12. System nach einem der Ansprüche 10 oder 11, das eine Benutzercomputervorrichtung (APP) umfasst, die konfiguriert ist zum Aufnehmen oder Speichern des mindestens einen fotografischen Bildes (IM) in einem Speicher und zum Kommunizieren des mindestens einen fotografischen Bildes in dem Importierungsschritt (IMP) zu dem Verarbeitungselement (PU).

13. Computerprogrammprodukt, das Anweisungen umfasst, die, wenn das Programm von einem Computer ausgeführt wird, den Computer veranlassen, das Verfahren nach einem der Ansprüche 1 bis 9 zu implementieren.

14. Computerlesbares Medium, das Anweisungen umfasst, die, wenn sie von einem Computer ausgeführt werden, den Computer veranlassen, das Verfahren nach einem der Ansprüche 1 bis 9 zu implementieren.

## Revendications

1. Procédé de détermination automatique au sein d'un système informatique d'au moins une couleur d'un produit cosmétique compatible avec une tenue d'un utilisateur, comprenant :
- une étape d'importation (IMP) d'au moins une image photographique (IM) contenant une représentation d'au moins une partie de la tenue de l'utilisateur ;
- une étape de segmentation (SEG) de ladite au moins une image (IM), pour identifier au moins une région d'intérêt (S1, S2, S3) dans la tenue ;
- une étape de détermination (DET) de la couleur de ladite au moins une région d'intérêt (S1, S2, S3) ; et
- une étape de génération (GEN) de ladite au moins une couleur compatible avec la tenue de l'utilisateur, sur la base de la couleur déterminée de ladite région d'intérêt (S1, S2, S3) en fonction d'une base de données (PAL1-PAL4) de couleurs compatibles ou d'une règle informatique (RGL, 11-71) d'obtention de couleurs compatibles.

2. Procédé selon la revendication 1, dans lequel l'étape de génération (GEN), réalisée en fonction d'une base de données de couleurs compatibles (PAL1-PAL4), comprend :
- une étape d'identification (ID), parmi des banques (PAL1-PAL4) de couleurs contenant un nombre fini de couleurs préalablement choisies pour être compatibles entre elles, de la banque (PAL1) de couleurs contenant la couleur la plus proche de la couleur de ladite région d'intérêt déterminée (S1, S2, S3) ; et
- une étape de sélection (SEL) de ladite au moins une couleur (c3.1, c3.2, c3.3) compatible avec la tenue parmi les couleurs de la banque identifiée (PAL1).

3. Procédé selon l'une des revendications 1 et 2, dans lequel l'étape de génération (GEN), réalisée en fonction d'une règle de calcul d'obtention des couleurs compatibles (RGL, 11-71), comprend :
- une étape de calcul (RGL) comprenant la conversion de la couleur de ladite région d'intérêt déterminée (S1, S2, S3) en un premier point d'un espace de couleur approprié, et l'application d'au moins une transformation mathématique (11-71) en ce point, ladite au moins une transformation mathématique (11-71) étant préalablement choisie pour obtenir en résultat au moins un deuxième point dudit espace dont la couleur étant compatible avec la couleur du premier point ; et
- une étape de sélection (SEL) de ladite au moins une couleur (c3.1, c3.2, c3.3) compatible avec la tenue parmi la ou les couleurs dudit au moins un deuxième point.

4. Procédé selon l'une des revendications 2 et 3, dans lequel ladite étape de sélection (SEL) comprend la sélection de ladite au moins une couleur compatible parmi les couleurs obtenues, ces couleurs obtenues appartenant à une famille (FAMa-FAMd) de couleurs sélectionnées par l'utilisateur.

5. Procédé selon l'une des revendications précédentes, dans lequel l'étape de génération (GEN) est mise en œuvre en utilisant un modèle d'apprentissage automatique (AI_GEN).

6. Procédé selon la revendication 5 prise en combinaison avec l'une des revendications 2 à 4, dans lequel le modèle d'apprentissage automatique (AI_GEN) est configuré pour adapter l'étape de sélection (SEL) en fonction de choix antérieurs de l'utilisateur sur des couleurs compatibles déterminées lors de mises en œuvre antérieures du procédé.

7. Procédé selon l'une des revendications 1 à 6, dans lequel l'étape de segmentation (SEG) est mise en œuvre automatiquement en utilisant un modèle d'apprentissage automatique (AI_TCOL).

8. Procédé selon l'une des revendications 1 à 6, dans lequel l'étape de segmentation (SEG) comprend une requête adressée à l'utilisateur pour identifier la position de ladite au moins une région d'intérêt dans l'image.

9. Procédé selon l'une des revendications précédentes, dans lequel l'étape de détermination (DET) comprend un traitement de couleur (TCOL) de ladite au moins une image (IMk) en utilisant un modèle d'apprentissage automatique (AI_TCOL) adapté pour corriger une déformation de la couleur de la représentation de ladite au moins une partie de la tenue de l'utilisateur du fait des conditions d'éclairage de la photographie et/ou du dispositif (CAM) ayant capturé la photographie.

10. Système informatique destiné à déterminer au moins une couleur d'un produit cosmétique compatible avec une tenue d'un utilisateur, comprenant :
- des moyens de communication (COM) configurés pour importer au moins une image photographique (IM) contenant une représentation d'au moins une partie de la tenue de l'utilisateur ;
- des moyens de traitement (PU) configurés pour :
-- segmenter (SEG) ladite au moins une image (IM), de façon à identifier au moins une région d'intérêt (S1, S2, S3) dans la tenue ;
-- déterminer (DET) la couleur de ladite au moins une région d'intérêt (S1, S2, S3) ; et
-- générer (GEN) ladite au moins une couleur compatible avec la tenue de l'utilisateur, sur la base de la couleur déterminée de ladite région d'intérêt (S1, S2, S3) en fonction d'une base de données (PAL1-PAL4) de couleurs compatibles ou d'une règle informatique (RGL, 11-71) d'obtention des couleurs compatibles.

11. Système selon la revendication 10, dans lequel les moyens de traitement (PU) étant en outre configurés pour mettre en œuvre les étapes de segmentation (SEG), l'étape de détermination (DET) et l'étape de génération (GEN) selon l'une des revendications 2 à 9.

12. Système selon l'une des revendications 10 ou 11, comprenant un dispositif informatique utilisateur (APP) configuré pour capturer ou mémoriser ladite au moins une image photographique (IM) et pour communiquer aux moyens de traitement (PU) ladite au moins une image photographique pendant ladite étape d'importation (IMP).

13. Produit de programme informatique comprenant des instructions qui, lorsque le programme est exécuté par un ordinateur, amènent ce dernier à mettre en œuvre le procédé selon l'une des revendications 1 à 9.

14. Support lisible par ordinateur, comprenant des instructions qui, lorsqu'elles sont exécutées par un ordinateur, amènent celui-ci à mettre en œuvre le procédé selon l'une des revendications 1 à 9.
